# EUROPEAN PATENT APPLICATION

(11) **EP 0 571 685 A1**
(43) Date of publication of application: **01.12.1993**
(21) Application number: 92610038.9
(22) Date of filing: 27.05.1992
(51) Int. Cl.: C07D 333/20, C07D 307/52, C07D 207/32, A61K 31/34, A61K 31/38, A61K 31/40

(54) **Aryloxyheteroarylpropylamines, their preparation and use**

(71) Applicant: NOVO NORDISK A/S, DK-2880 Bagsvaerd (DK)
(72) Inventor: Jakobsen, Palle, DK-3500 Vaerlose (DK); Kanstrup, Anders, DK-2830 Virum (DK); Lundbeck, Jane Marie, DK-2600 Glostrup (DK)

(57) **Abstract**

The present invention relates to aryloxyheteroarylpropylamines of formula I 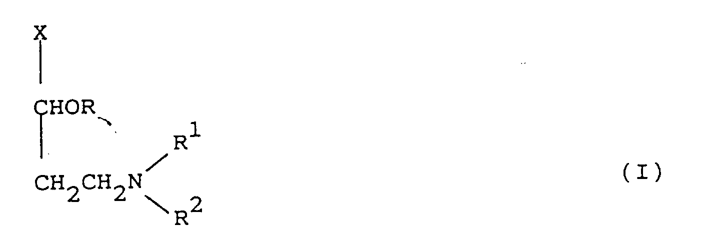
wherein
X is 2- or 3-thienyl-, furanyl or pyrrolyl, all of which are unsubstituted or optionally substituted; and
R is 3,4-methylenedioxyphenyl, aryl or heteroaryl, all of which are unsubstituted or optionally substituted; and
R¹ and R² are hydrocarbyl substituents, containing 1 to 11 carbon atoms, all of which are unsubstituted or optionally substituted; or hydrogen; or
R¹ and R² together form a 5, 6 or 7 membered ring containing at least one nitrogen atom, or optionally contains two nitrogen atoms, one or two oxygen atom(s) or one or two sulfur atom(s) or a combination thereof, which ring is unsubstituted or optionally substituted,
a method of preparing the same and to pharmaceutical compositions comprising the compounds.
The compounds of the invention have activity against calcium overload in brain cells, and are useful in the treatment of anoxia, traumatic injury, ischemia, migraine, epilepsy, Parkinson's disease, Alzheimer's disease and other neurodegenerative diseases.

## Description

The present invention relates to therapeutically active aryloxyheteroarylpropylamines, a method of preparing the same and to pharmaceutical compositions comprising the compounds. The novel compounds are useful in the treatment of anoxia, traumatic injury, ischemia, migraine, epilepsy, Parkinson's disease, Alzheimer's disease and other neurodegenerative diseases.

It is well known that accumulation of calcium in the brain cells (calcium overload) is seen after periods of uncontrolled hyperactivity in the brain, such as after convulsions, migraine, anoxia and ischemia. As the concentration of calcium in the cells is of vital importance for the regulation of cell function, an uncontrolled high concentration of the cell calcium will lead to, or indirectly cause the symptoms and possibly also the degenerative changes combined with the above diseases.

Therefore, calcium overload blockers selective for brain cells will be useful in the treatment of anoxia, traumatic injury, ischemia, migraine, epilepsy, Parkinson's disease, Alzheimer's disease and other neurodegenerative diseases.

Well known calcium antagonists such as nifedipine, verapamil and diltiazem have activity against peripheral calcium uptake, e.g. in blood vessels and the heart, however, they have shown only very low activity against calcium overload in brain cells.

Accordingly it is an object of the invention to provide novel compounds having activity against calcium overload in brain cells.

The novel compounds of the invention are aryloxyheteroarylpropylamines of formula I 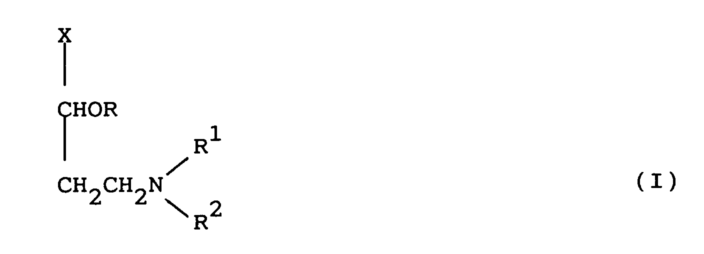
wherein
X is 2- or 3-thienyl, 2- or 3-furanyl, 2- or 3-pyrrolyl, all of which are unsubstituted or optionally substituted with one or more C₁₋₆-alkyl, C₁₋₆-alkanoyl, C₃₋₅-alkenyl, C₁₋₆-alkoxy, cyano, halogeno, halogenoalkyl; and
R is 3,4-methylenedioxyphenyl, aryl or heteroaryl, all of which are unsubstituted or optionally substituted with one or more cyano, halogeno, C₁₋₆-alkyl, C₁₋₆-alkoxy, C₂₋₆-alkenyl, trifluoromethyl, C₃₋₅-alkylene, aryloxy, aralkoxy or C₁₋₆-alkylthio; and
R¹ and R² are C₁₋₁₀-alkyl, C₃₋₇-cycloalkyl, c₂₋₁₀-alkenyl, C₃₋₆-cycloalkyl-C₁₋₅-alkyl, all of which are unsubstituted or substituted with C₁₋₅-alkoxy or cyano; or
R¹ and R² together form a 5, 6 or 7 membered ring containing at least one nitrogen atom, or which optionally contains two nitrogen atoms, one or two oxygen atom(s) or one or two sulphur atom(s) or a combination thereof, which ring is unsubstituted or optionally substituted with C₁₋₄-alkyl, C₁₋₄-alkoxy or aryl, or a salt thereof with a pharmaceutically acceptable acid;
provided however that R¹ and R² are not H or C₁₋₃-alkyl when X is a thienyl group or R¹ and R² are methyl when X is halothienyl, C₁₋₄-alkylthienyl or furanyl and the R-aryl group is substituted with halogen, C₁₋₄-alkyl, C₁₋₃-alkoxy or trifluoromethyl.

Alkyl is intended to mean straight or branched alkyl radicals.

Examples of such salts include inorganic and organic acid addition salts such as hydrochloride, hydrobromide, sulphate, phosphate, acetate, fumarate, maleate, citrate, lactate, tartrate, oxalate, or similar pharmaceutically acceptable inorganic or organic acid addition salts.

The invention also relates to a method of preparing the above mentioned compounds. These methods comprise
a) reacting a compound of formula II 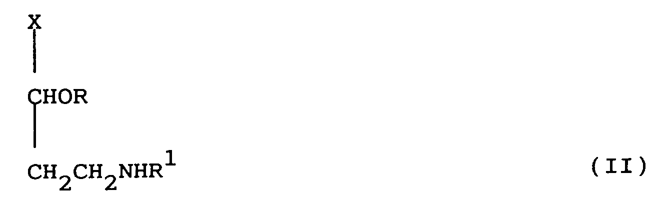 wherein X, R¹ and R have the meanings defined above, with a compound of formula R²-Y, wherein Y is a leaving group such as halogen and R² has the meaning defined above; or
b) reacting a compound of formula III 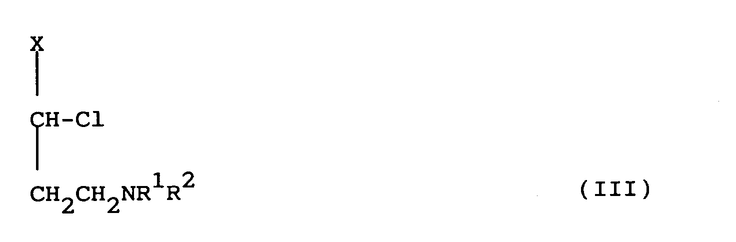 with a compound of formula IV

   ROH (IV)

   wherein R, R¹ and R² have the meaning defined above; or
c) preparing a compound of formula III from the corresponding hydroxy compound by means of SOCl₂_{'} where the hydroxy compound is prepared by a NaBH₄ reduction of the corresponding oxo-compound, which again is prepared by a Mannich reaction; or
d) preparing a compound of formula II by demethylating a compound of formula I by means of ROCOCl, especially R as -CHClCH₃_{'} vinyl and CH₂CCl₃ are preferable; or
e) reacting a compound of formula V 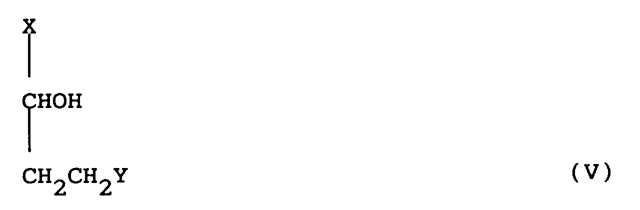 with a compound of formula R¹NHR² wherein X, R¹ and R² have the meanings defined above, and Y is a leaving group such as halogen; or
f) reacting a compound of formula VI 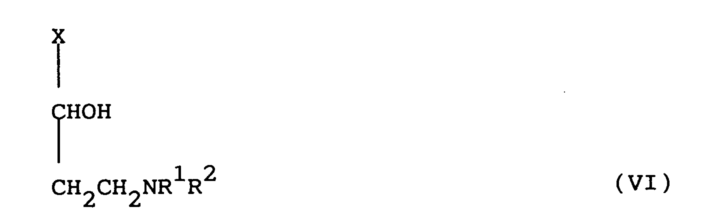 with a compound of formula 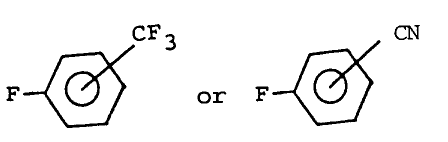 wherein X, R¹ and R² have the meanings defined above; or
g) reacting a compound of formula VI 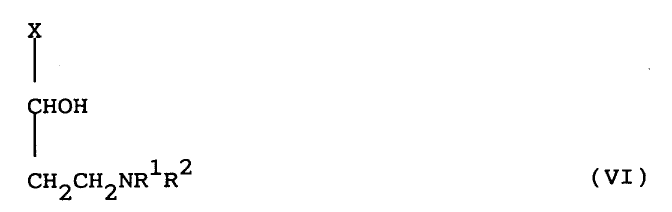 with a compound of formula IV

   ROH (IV)

   by means of Ph₃P and (EtOCON)₂ (Mitsunobu, O. Synthesis 1981 p.1); or
h) preparation of a compound of formula I by modification of the substituent in the X-group of a compound of formula I by known chemical methods.

These methods comprise reduction of NO₂-groups, alkylation of amino groups, hydrolysis of CN-groups giving acids or amides, N-alkylation of carboxamides, aromatic substitution of halogeno compounds or sulfonylation.

The compounds of the present invention possess an asymmetric carbon atom and can therefore exist in the individual R and S isomers as well as the racemic mixture. The present invention relates to both the racemates and the individual optically active stereo isomers.

Specific compounds within the scope of the present invention include the following and pharmaceutically acceptable salts thereof:
1-(3-(5-Bromo-2-thienyl)-3-(4-trifluoromethylphenoxy)propyl)-4-methylpiperazine,
1-(3-(5-Bromo-2-thienyl)-3-(4-trifluoromethylphenoxy)propyl)-4-phenylpiperazine,
1-(3-(5-chloro-2-thienyl)-3-(3-trifluoromethylphenoxy)propyl)-4-phenylpiperazine,
1-(3-(2,5-dimethyl-3-thienyl)-3-(4-trifluoromethylphenoxy)propyl)piperidine,
1-(3-(2,5-dimethyl-3-thienyl)-3-(2-trifluoromethylphenoxy)propyl)piperidine,
1-(2,5-dimethyl-3-thienyl)-1-(4-trifluoromethylphenoxy)-3-morpholinopropane,
1-(3-thienyl)-1-(4-trifluoromethylphenoxy)-3-morpholinopropane,
1-(5-chloro-2-thienyl)-1-(4-phenylphenoxy)-3-morpholinopropane,
N,N-dibutyl-3-(3-chloro-2-thienyl)-3-(4-trifluoromethylphenoxy)propanamine,
N-butyl-N-cyclopropylmethyl-3-(5-chloro-2-thienyl)-3-(3-trifluoromethylphenoxy)propanamine,
1-(3-(2-thienyl)-3-(4-trifluoromethylphenoxy)propyl)-4-phenylpiperazine,
1-(3-(3-thienyl)-3-(4-trifluoromethylphenoxy)propyl)-4-ethylpiperazine,
1-(3-(3-thienyl)-3-(4-phenoxyphenoxy)propyl)-4-propylpiperazine,
1-(3-(2-thienyl)-3-(4-isopropylphenoxy)propyl)-4-phenylpiperazine,
1-(3-(3-thienyl)-3-(3-trifluoromethylphenoxy)propyl)piperidine,
1-(3-(3-thienyl)-3-(4-trifluoromethylphenoxy)propyl)piperidine,
1-(3-(3-thienyl)-3-(4-cyanophenoxy)propyl)piperidine.

The pharmacological properties of the compounds of the invention can be illustrated by determining their capability to inhibit calcium uptake into brain synaptosomes.

### PRINCIPLE

Depolarization of neuronal membranes leads to an opening of socalled "voltage operated calcium channels" (VOC) in the membranes which allows a massive influx of calcium from the extracellular space. A crude synaptosomal preparation (socalled P₂ fraction) contains small vesicles surrounded by neuronal membrane and it is possible in such a preparation to study a depolarization-induced opening of VOC. In the present model ⁴⁵Ca influx is induced in the synaptosomes by depolarization with elevated potassium concentrations, and the effect of test substances on this stimulated uptake is studied (Nachshen, D.A. and Blaustein, M.P., Mol. Pharmcol., 16, 579 (1979)).

### ASSAY

A male Wistar rat is decapitated and the cerebral cortex removed and homogenized in 10 ml. of ice-cold 0.32 M sucrose using a glass homogenizer with a teflon pestle. All subsequent steps for isolation of synaptosomes are done at 0-4°C. The homogenate is centrifuged at 1000 x g for 10 min and the resulting supernatant is re-centrifuged at 18000 x g for 20 min. This pellet (P₂) is resuspended in 0.32 M sucrose (5 ml per g of original tissue) with a teflon pestle.

Aliquots (0.050 ml) of this crude synaptosomal suspension are added to glass tubes containing 0.625 ml of NaCl buffer (136 mM NaCl, 4 mM KCl, 0.35 mM CaCl₂_{'} 1.2 mM MgCl₂, 20 mM Tris HCl, 12 mM glucose, pH 7.4) and 0.025 ml of various drug solutions in 48% Ethanol. The tubes are pre-incubated for 30 min on ice and then for 6 min at 37°C in a water bath.

The uptake is immediately initiated by adding 0.4 ml of ⁴⁵CaCl₂ (specific activity = 29-39 Ci/g; 0.5 µCi/assay), in 145 mM NaCl for non-depolarized samples and in 145 mM KCl for depolarized samples. The incubation is continued for 15 s.

The uptake is terminated by rapid filtration through GF-C glass fiber filters which are washed three times with 5 ml of a cold solution containing 145 mM KCl, 7 mM EGTA and 20 mM Tris HCl, pH 7.4. The amount of radioactivity on the filter disc is determined by liquid scintillation spectrometry.

### TEST PROCEDURE

Test substances are dissolved in 10 ml of 48% ethanol at a concentration of 0.44 mg/ml. Dilution are made in 48% ethanol to give final concentrations of 0.1, 0.3, 1, 3 and 10 µg/ml. Experiments are performed in triplicate. Controls for depolarized and nondepolarized samples are included in the assay and test substances are only tested in depolarized samples. 25-75% inhibition of stimulated uptake must be obtained before calculating the IC₅₀ value.

### RESULTS

The test value will be given as IC₅₀ (the concentration (µg/ml) of test substance which inhibit 50% of stimulated uptake of ⁴⁵Ca (uptake in depolarized samples corrected for basal uptake in nondepolarized samples )). The IC₅₀ value is estimated from dose response curves.

Test results obtained by testing some compounds of the present invention will appear from the following table 1

**TABLE 1**

| Compound | IC₅₀ (µg/ml) |
|---|---|
| 3 | 6.6 |
| 4 | 3.8 |
| 11 | 10.3 |
| 15 | 2.4 |
| 16 | 12.0 |
| 17 | 3.0 |
| 18 | 7.3 |
| 19 | 4.4 |
| 21 | 5.9 |
| 22 | 4.4 |
| 23 | 8.6 |
| 24 | 5.8 |
| 27 | 13 |
| * Nifedipine | 26 |
| * Verapamil | 16 |
| * Diltiazem | > 90 |
| * Flunarizine | 20 |

| | |
|---|---|
| * well known calcium antagonists. | |

The compounds of the invention, together with a conventional adjuvant, carrier, or diluent, and if desired in the form of a pharmaceutically acceptable acid addition salt thereof, may be placed into the form of pharmaceutical compositions and unit dosages thereof, and in such form may be employed as solids, such as tablets of filled capsules, or liquids, such as solutions, suspensions, emulsions, elixirs, or capsules filled with the same, all for oral use, in the form of suppositories for rectal administration; or in the form of sterile injectable solutions for parenteral use (including subcutaneous administration and infusion). Such pharmaceutical compositions and unit dosage forms thereof may comprise conventional ingredients in conventional proportions, with or without additional active compounds or principles, and such unit dosage forms may contain any suitable effective calcium overload blocking amount of the active ingredient commensurate with the intended daily dosage range to be employed. Tablets containing ten (10) milligrams of active ingredient or, more broadly, ten (10) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets, dragees, or capsules having talc and/or a carbohydrate carrier or binder or the like, the carrier preferably being lactose and/or corn starch and/or potato starch, are particularly suitable for oral application. A syrup, elixir of the like can be used in cases where a sweetened vehicle can be employed.

Generally, the compounds of this invention are dispensed in unit form comprising 0.05-100 mg in a pharmaceutically acceptable carrier per unit dosage.

The dosage of the compounds according to this invention is 0.1-300 mg/day, preferably 10-100 mg/day, when administered to patients, e.g. humans, as a drug.

A typical tablet which may be prepared by conventional tabletting techniques contains:

| | |
|---|---|
| Active compound | 5.0 mg |
| Lactosum | 67.8 mg Ph.Eur. |
| Avicel™ | 31.4 mg |
| Amberlite™IRP 88 | 1.0 mg |
| Magnesii stearas | 0.25 mg Ph.Eur. |

Due to te high calcium overload blocking activity, the compounds of the invention are extremely useful in the treatment symptoms related to an accumulation of calcium in brain cells of mammals, when administered in an amount effective for blocking activity of compounds of the invention includes both activity against anoxia, traumatic injury, ischemia, migraine, epilepsy, Parkinson's disease, Alzheimer's disease and other neurodegenerative diseases. The compounds of the invention may accordingly be administered to a subject, e.g., a living animal body, including a human, in need of a calcium overload blocker, and if desired in the form of a pharmaceutically acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultanously, or together with a pharmaceutically acceptable carrier or diluent, especially and preferably in the form of a pharmaceutical composition thereof, whether by oral, rectal, or parenteral (including subcutanous) route, in an effective calcium overload blocking amount, and in any event an amount which is effective for the treatment of anoxia, traumatic injury, ischemia, migraine, epilepsy, Parkinson's disease, Alzheimer's disease and other neurodegenerative diseases due to their calcium overload blocking activity. Suitable dosage ranges are 1-200 milligrams daily, 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples.

### EXAMPLE 1

### 3-Butylamino-1-(2,5-dimethyl-3-thienyl)propanon (1)

Prepared from 3-acetyl-2,5-dimethylthiophene (4.3 g), butanamine (3.9 ml) and paraformaldehyde (1.2 g) by heating in ethanol (30 ml) acidified with conc. HCl. (5 ml) following the normal Mannich reaction procedure, yield of 15 g of (1), identified by ¹H NMR.

### 3-Butylamino-1-(2,5-dimethyl-3-thienyl)-1-propanol (2)

Compound (1) (5 g) was reduced without further purification with NaBH₄ (2.5 g) in ethanol by stirring in ethanol at room temperature overnight. The reaction mixture was evaporated and extracted with NaOH/ether. The ether phases were combined and evaporated to dryness giving 1.5 g of (2), identified by ¹H NMR. The crude product was used without further purification.

### N-butyl-3-(4-cyanophenoxy)-3-(2,5-dimethyl-3-thienyl)propanamine, oxalate (3)

Prepared from compound (2) (0.55 g) and 4-fluorobenzonitrile (0.5 g) in dry DMF (25 ml) under N₂. 0.45 g potassium tert.butoxide was added under cooling in ice and subsequently the mixture was heated to 70^{o}C and refluxed overnight. The reaction mixture was rinsed up by addition of water followed by extraction with ether. The etheral layers were evaporated and the residue was purified on silica gel using CH₂Cl₂/CH₃OH 9/1 as eluent. Yield 0.27 g oil which was precipitated as the oxalate from acetone solution. M.p. 162.5^{o}C.

### N-butyl-3-(2,5-dimethyl-3-thienyl)-3-(4-trifluoromethylphenoxy)propanamine, oxalate (4)

Prepared from compound (2) 0.6 g and 4-fluorobenzoetrifluoride (0.7 g) using the method described for the preparation of compound (3). Yield after purification on silica gel 0.43 g oil which was precipitated from acetone as the oxalate. M.p. 172.4^{o}C.

### 3-(3-cyanophenoxy)-N,N-dimethyl-3-(2,5-dimethyl-3-thienyl)propanamine, oxalate (5)

3-(N,N-dimethylamino)-1-(2,5-dimethyl-3-thienyl)-1-propanol (6) was prepared by NaBH₄ reduction of the Mannich product - analogous to what is described for compound (2). Compound (6) (1 g) was treated with 3-fluorobenzonitrile (1 ml) and potassium tert.butoxide (1 g) as described for compound (3). The compound was isolated as the oxalate after rinse up as described for (3). Yield 0.7 g. M.p. 172^{o}C^{.}

### N,N-dimethyl-3-(4-isopropylphenoxy)-3-(2,5-dimethyl-3-thienyl)propanamine, oxalate (7)

Prepared from compound (6) (1 g), 4-isopropylphenol (0.54 g) and triphenylphosphine (1.6 g) in THF (25 ml). Diethylazodicarboxylate (DEAD) (0.7 ml) was added while cooling on ice. The reaction mixture was kept in a N₂-atmosphere and stirred at room temperature overnight. The reaction mixture was evaporated, extracted with ether/OH⁻, the etheral layer was cooled, which caused precipitation of Ph₃PO. This was removed by filtration and the filtrate was rinsed on silica gel twice using 1) CH₂Cl₂/CH₃OH 9/1 and 2) ethyl acetate followed by methanol as eluent. Yield of (7) 0.27 g which was precipitated as the oxalate from acetone solution. M.p. 160-162^{o}C.

### N,N-dimethyl-3-(2,5-dimethyl-3-thienyl)-3-(3-trifluoromethylphenoxy)propanamine, oxalate (8)

Prepared from (6) (1 g) and 3-trifluoromethylphenol (0.5 ml) by the Mitsunobu reaction described for (7). Only one silica gel rinse up with CH₂Cl₂/CH₃OH 9/1 was required. Yield 0.35 g of the oxalate, m.p. 167.1-168.7^{o}C.

### N,N-dimethyl-3-(2,5-dimethyl-3-thienyl)-3-(4-phenylphenoxy)propanamine, oxalate (9)

Preparation by the Mitsunobu reaction from (6) (1 g) and 4-phenylphenol (0.8 g) as described for compound (8). The product was purified twice on a silica gel column using CH₂Cl₂/CH₃OH (9/1) as eluent. Yield 0.47 g. Precipitated as oxalate from acetone. M.p. 183.4 - 185.5^{o}C.

### N,N-dimethyl-3-(2,5-dimethyl-3-thienyl)-3-(4-phenoxyphenoxy)propanamine, oxalate (10)

Prepared by the Mitsunobu reaction from 4-phenoxyphenol (0.9 g) and (6) (1 g) using the rinse up procedure described for compound (8). Yield of oxalate 0.37 g, m.p. 115.5 - 116.6^{o}C.

### EXAMPLE 2

### N,N-Dimethyl-3-(2,4-dimethyl-3-furyl)-3-(4-trifluoromethylphenoxy)propaneamine, oxalate (11)

3-dimethylamino-1-(2,4-dimethyl-3-furyl)-1-propanol (12) was prepared analogous to what is described for (6) and identified by ¹H and ¹³C NMR. (12) (5 g) and NaH (1.5 g) were heated in dry DMF (50 ml) to 60^{o}C. 4-Fluorobenzotrifluoride (4.5 ml) was added and the mixture refluxed for 2 h. The reaction mixture was extracted with 4 M NaOH/ether, the etheral layer evaporated and the residue purified on a silica gel column giving 2.6 g oil which was identified as (11) by ¹H and ¹³C NMR. 0.55 g of this oil was precipitated as the oxalate giving 0.62 g colourless crystals, m.p. 137.2^{o}C.

### EXAMPLE 3

### 1-(5-chloro-2-thienyl)-3-(1-piperidyl)propan-1-on (13)

Prepared by a Mannich reaction from 2-acetyl-5-chlorothiophene (5 g), paraformaldehyde (1 g) and piperidine (10 ml), yield 5.3 g after isolation of the free base.

This was in turn reduced by means of NaBH₄ (4 g) in ethanol (75 ml) at room temperature by stirring overnight. Yield after rinse up 3.5 g of 1-(5-chloro-2-thienyl)-3-(1-piperidinyl)-1-propanol) (14) identified by ¹H and ¹³C NMR.

### 1-(3-(5-chloro-2-thienyl)-3-(4-trifluoromethylphenoxy)propyl)piperidine, oxalate (15)

(14) (1 g), 4-fluorobenzotrifluoride (0.65 g) and potassium tert.butoxide (0.45 g) were reacted in dry DMF (50 ml) as described for (3). Reaction time 3 days at room temperature. Rinse up on silica gel followed by precipitation as the oxalate gave 0.74 g crystals, m.p. 151-152^{o}C.

### 1-(3-(5-chloro-2-thienyl)-3-(3-cyanophenoxy)propyl)piperidine, oxalate (16)

Preparation from (14) (0.8 g) and 3-fluorobenzonitrile (0.43 g) as described for (15) gave 0.66 g oxalate. M.p. 126-128^{o}C.

### 1-(3-(5-chloro-2-thienyl)-3-(2-trifluoromethylphenoxy)propyl)piperidine, oxalate (17)

Preparation from 0.8 g (14) and 2-fluorobenzotrifluoride (0.5 ml) as described for (15) gave 0.17 g oxalate. M.p. 153^{o}C.

### 1-(3-(5-chloro-2-thienyl)-3-(3-trifluoromethylphenoxy)propyl)piperidine, oxalate (18)

0.9 g (14), 0.78 g triphenylphosphine, 0.485 g 3-trifluoromethylphenol and 0.49 ml diethylazodicarboxylate were reacted in dry THF (25 ml) in a Mitsunobu reaction. After precipitation of excess Ph₃PO with ether the filtrate was purified on silica gel using petrol ether/triethylamine (15/1) as eluent. The pure fraction of (18) was precipitated as the oxalate giving 0.2 g crystals, m.p. 167-170^{o}C.

### EXAMPLE 4

### 3-(5-Bromo-2-thienyl)-N,N-dimethyl-3-(4-(methylthio)phenoxy)propanamine, oxalate (19)

1-(5-bromo-2-thienyl)-N,N-dimethylamino propan-1-ol (20) was prepared from the corresponding oxo compound prepared from 2-acetyl-5-bromothiophene, paraformaldehyde and dimethyl ammonium chloride using the well known Mannich reaction conditions. (20) (1 g), triphenylphosphine (0.97 g), 4-(methylthio)phenol (0.53 g) and DEAD (0.57 ml) were reacted in dry THF (25 ml) as described for (7). The crude product was purified 3 times on silica gel column using CH₂Cl₂/CH₃OH (9/1) as eluent. Yield 0.43 g oil which was precipitated from acetone solution as the oxalate. M.p. 140-141^{o}C.

### 3-(5-Bromo-2-thienyl)-N,N-dimethyl-3-(4-phenoxyphenoxy)propanamine, oxalate (21)

Preparation from (20) (1 g) and 4-phenoxyphenol (0.69 g) as described for (19) gave 0.35 g of the oxalate. M.p. 145-146^{o}C.

### 3-(5-Bromo-2-thienyl)-N,N-dimethyl-3-(4-phenylphenoxy)propanamine, oxalate (22)

Preparation from (20) (1 g) and 4-phenylphenol(0.63 g) as described for (19) gave the oxalate 0.35 g. M.p. 161^{o}C.

### EXAMPLE 5

### N,N-dimethyl-3-(2-thienyl)-3-(4-trifluoromethylphenoxy)propanamine, oxalate (23)

3-(N,N-dimethylamino)-1-(2-thienyl)propan-1-ol (5 g) was treated with 4-fluorobenzotrifluoride (4.9 g) and potassium tert.butoxide (3.3 g) in dry DMF (50 ml) as described for (3). Yield 5 g. Precipitation as the oxalate. M.p. 127-128^{o}C.

### 1-(3-(2-Thienyl)-3-(4-trifluoromethylphenoxy)propyl)piperidine, oxalate (24)

Preparation from 3-(1-piperidyl)-1-(2-thienyl)propan-1-ol (25) (0.95 g), 4-fluorobenzotrifluoride (0.5 ml) and potassium tert.butoxide (0.45 g) in dry DMF (50 ml) as described for (3), yield of the oxalate 0.48 g, m.p. 143.1^{o}C. Compound (25) was prepared in the normal way through a Mannich reaction with subsequent NaBH₄ reduction.

### 1-(3-(2-Thienyl)-3-(3-cyanophenoxy)propyl)piperidine, oxalate (26)

Preparation from (25) (0.6 g) and 3-fluorobenzonitrile (0.3 ml) as described for (24) gave 0.26 g of the oxalate, m.p. 162-164^{o}C.

### EXAMPLE 6

### N,N-dimethyl-3-(3-thienyl)-3-(4-trifluoromethylphenoxy)propanamine, hydrochloride (27)

Prepared from 3-dimethylamino-1-(2-thienyl)-propan-1-ol (5 g) and 4-fluorobenzotrifluoride (4,5 ml) by means of NaH (1.5 g) in dry DMF (50 ml) as described for (11). Purified on a silica gel column using ethyl acetate/triethylamine (10/1) as eluent. Yield 6.0 g. Precipitation as the hydrochloride. M.p. 125.8^{o}C.

### N-butyl-N-cyclopropylmethyl-3-(3-thienyl)-3-(4-trifluoromethylphenoxy)propanamine, oxalate (28)

3-(N-Butyl-N-cyclopropylmethylamino)-1-(3-thienyl)propan-1-ol (29) (0.4 g) was heated with 4-fluorobenzotrifluoride (0.25 ml) and potassium tert.butoxide (0.25 g) in dry DMF (25 ml) as described for (3). After purification on silica gel and precipitation as the oxalate the yield was 0.2 g, m.p. 116-118^{o}C.

### N-butyl-N-cyclopropylmethyl-3-(4-cyanophenoxy)-3-(3-thienyl)propanamine, oxalate (30)

Preparation from (29) (0.4 g) and 4-fluorobenzonitrile (0.23 ml) as described for (28). Yield of the oxalate 0.21 g, m.p. 128.3-128.8^{o}C.

### EXAMPLE 7

### 1-(5-Chloro-2-thienyl)-3-(4-methyl-1-piperazinyl)propan-1-on (31)

Prepared in a Mannich type reaction between 2-acetyl-5-chlorothiophen (5 g), paraformaldehyde (1 g), N-methylpiperazine (10 ml) and conc. HCl in abs. ethanol. This resulted in a crude product (15.4 g) which was contaminated with N-methyl-piperazine hydrochloride, identification by ¹H and ¹³C NMR. The mixture was reduced with NaBH₄ (4 g) in ethanol (200 ml) without further purification by stirring at room temperature for 72 h. Rinse up gave 1-(5-chloro-2-thienyl)-3-(4-methyl-1-piperazinyl)propan-1-ol (32) as an oil 4.2 g, identified by ¹H and ¹³C NMR.

### 1-(3-(5-Chloro-2-thienyl)-3-(4-trifluoromethylphenoxy)propyl)-4-methylpiperazine, fumarate (33)

(32) (2 g), 4-fluorobenzotrifluoride (1 ml) and potassium tert.butoxide (1 g) were stirred at room temperature in dry DMF (50 ml) and rinsed up as described for (3). After purification on column the yield was 0.8 g oil. Precipitation as the fumarate gave 0.4 g. M.p. 195^{o}C. d.

### 1-(3-(5-Chloro-2-thienyl)-3-(4-(methylthio)phenoxy)propyl)- 4-methylpiperazine, fumarate (34)

Preparation from (32) (2.0 g) and 4-(methylthio)phenol (1.1 g) by the Mitsunobu reaction gave 0.75 g oil. Precipitation as the fumarate gave 0.43 g. M.p. 195^{o}C. d.

## Claims

1. A compound of formula I 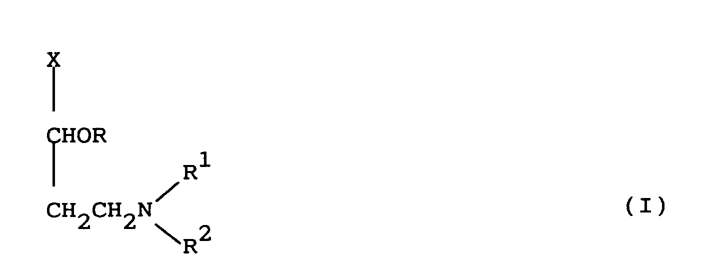 wherein
X is 2- or 3-thienyl, 2- or 3-furanyl, 2- or 3-pyrrolyl, all of which are unsubstituted or optionally substituted with one or more C₁₋₆-alkyl, C₁₋₆-alkanoyl, C₃₋₅-alkenyl, C₁₋₆-alkoxy, cyano, halogeno, halogenoalkyl; and
R is 3,4-methylenedioxyphenyl, aryl or heteroaryl, all of which are unsubstituted or optionally substituted with one or more cyano, halogeno, C₁₋₆-alkyl, C₁₋₆-alkoxy, C₂₋₆-alkenyl, trifluoromethyl, C₃₋₅-alkylene, aryloxy, aralkoxy or C₁₋₆-alkylthio; and
R¹ and R² are C₁₋₁₀-alkyl, C₃₋₇-cycloalkyl, C₂₋₁₀-alkenyl, C₃₋₆-cycloalkyl-C₁₋₅-alkyl, all of which are unsubstituted or substituted with C₁₋₅-alkoxy or cyano; or
R¹ and R² together form a 5, 6 or 7 membered ring containing at least one nitrogen atom, or which optionally contains two nitrogen atoms, one or two oxygen atom(s) or one or two sulphur atom(s) or a combination thereof, which ring is unsubstituted or optionally substituted with C₁₋₄-alkyl, C₁₋₄-alkoxy or aryl, or a salt thereof with a pharmaceutically acceptable acid;
provided however that R¹ and R² are not H or C₁₋₃-alkyl when X is a thienyl group or R¹ and R² are methyl when X is halothienyl, C₁₋₄-alkylthienyl or furanyl and the R-aryl group is substituted with halogen, C₁₋₄-alkyl, C₁₋₃-alkoxy or trifluoromethyl.

2. The compound according to claim 1 selected from
N-butyl-3-(4-cyanophenoxy)-3-(2,5-dimethyl-3-thienyl)propanamine,
N-butyl-3-(2,5-dimethyl-3-thienyl)-3-(4-trifluoromethylphenoxy)propanamine,
3-(3-cyanophenoxy)-N,N-dimethyl-3-(2,5-dimethyl-3-thienyl)propanamine,
N,N-dimethyl-3-(4-isopropylphenoxy)-3-(2,5-dimethyl-3-thienyl)propanamine,
N,N-dimethyl-3-(2,5-dimethyl-3-thienyl)-3-(3-trifluoromethylphenoxy)propanamine,
N,N-dimethyl-3-(2,5-dimethyl-3-thienyl)-3-(4-phenylphenoxy)propanamine,
N,N-dimethyl-3-(2,5-dimethyl-3-thienyl)-3-(4-phenoxyphenoxy)propanamine,
N,N-dimethyl-3-(2,4-dimethyl-3-furyl)-3-(4-trifluoromethylphenoxy)propanamine,
1-(3-(5-chloro-2-thienyl)-3-(4-trifluoromethylphenoxy)propyl)piperidine,
1-(3-(5-chloro-2-thienyl)-3-(3-cyanophenoxy)propyl)piperidine,
1-(3-(5-chloro-2-thienyl)-3-(2-trifluoromethylphenoxy)propyl)piperidine,
1-(3-(5-chloro-2-thienyl)-3-(3-trifluoromethylphenoxy)propyl)piperidine,
3-(5-bromo-2-thienyl)-N,N-dimethyl-3-(4-(methylthio)phenoxy)propanamine,
3-(5-bromo-2-thienyl)-N,N-dimethyl-3-(4-phenoxyphenoxy)propanamine,
3-(5-bromo-2-thienyl)-N,N-dimethyl-3-(4-phenylphenoxy)propanamine,
N,N-dimethyl-3-(2-thienyl)-3-(4-trifluoromethylphenoxy)propanamine,
1-(3-(2-thienyl)-3-(4-trifluoromethylphenoxy)propyl)piperidine,
1-(3-(2-thienyl)-3-(3-cyanophenoxy)propyl)piperidine,
N,N-dimethyl-3-(3-thienyl)-3-(4-trifluoromethylphenoxy)propanamine,
N-butyl-N-cyclopropylmethyl-3-(3-thienyl)-3-(4-trifluoromethylphenoxy)propanamine,
N-butyl-N-cyclopropylmethyl-3-(4-cyanophenoxy)-3-(3-thienyl)propanamine,
1-(3-(5-chloro-2-thienyl)-3-(4-trifluoromethylphenoxy)propyl)-4-methylpiperazine,
1-(3-(5-chloro-2-thienyl)-3-(4-(methylthio)phenoxy)propyl)-4-methylpiperazine,
or a pharmaceutically acceptable salt thereof.

3. A method of preparing a compound according to claim 1 characterized in
a) reacting a compound of formula II 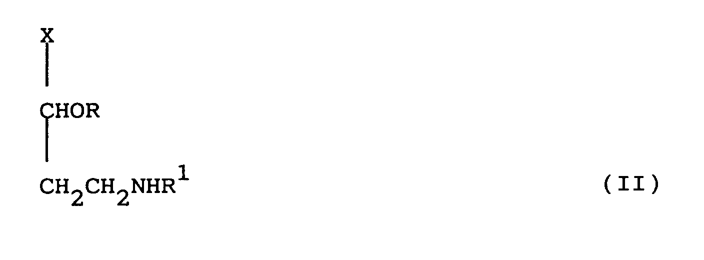 wherein X, R¹ and R have the meanings defined above, with a compound of formula R²-Y, wherein Y is a leaving group such as halogen and R² has the meaning defined above; or
b) reacting a compound of formula III 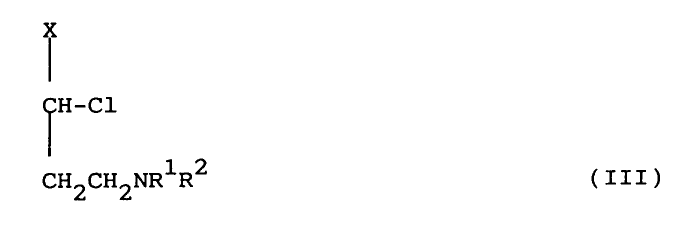 with a compound of formula IV
ROH (IV)
wherein R, R¹ and R² have the meaning defined above; or
c) preparing a compound of formula III from the corresponding hydroxy compound by means of SOCl₂, where the hydroxy compound is prepared by a NaBH₄ reduction of the corresponding oxo-compound, which again is prepared by a Mannich reaction; or
d) preparing a compound of formula II by demethylating a compound of formula I by means of ROCOCl, especially R as -CHClCH₃_{'} vinyl and -CH₂CCl₃ are preferable; or
e) reacting a compound of formula V 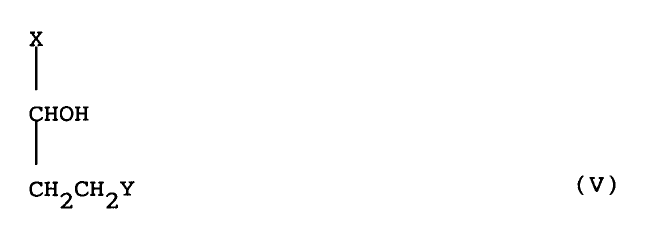 with a compound of formula R¹NHR² wherein X, R¹ and R² have the meanings defined above, and Y is a leaving group such as halogen; or
f) reacting a compound of formula VI 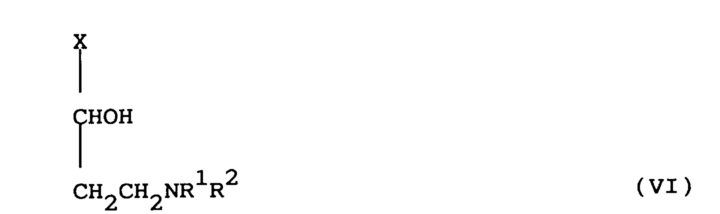 with a compound of formula 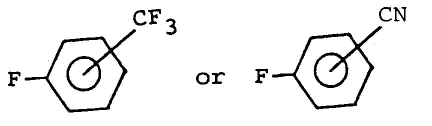 wherein X, R¹ and R² have the meanings defined above; or
g) reacting a compound of formula VI 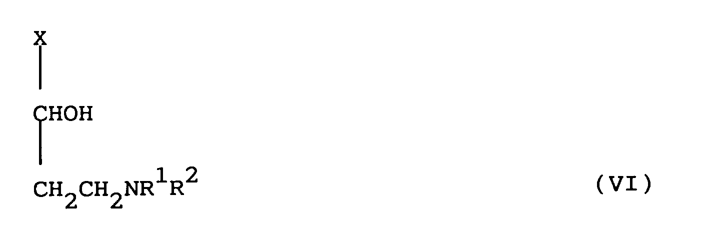 with a compound of formula IV
ROH (IV)
by means of Ph₃P and (EtOCON)₂; or
h) preparation of a compound of formula I by modification of the substituent in the X-group of a compound of formula I by known chemical methods.

4. A pharmaceutical composition comprising a compound of claim 1 or a salt thereof with a pharmaceutically acceptable acid together with a pharmaceutically acceptable carrier or diluent.

5. A pharmaceutical composition suitable for use in preventing calcium overload in brain cells of mammals, including humans, comprising an amount of a compound of claim 1, which is effective for inhibiting calcium uptake into brain cells together with a pharmaceutically acceptable carrier or diluent.

6. The pharmaceutical composition according to claim 4 or 5 wherein it is in the form of an oral dosage unit containing 1-100 mg of the active compound.

7. A method of treating calcium overload in brain cells of mammals, including humans, comprising administering a calcium overload blocking amount of a compound according to claim 1.

8. A method of treating calcium overload in brain cells of mammals, including humans, comprising administering a pharmaceutical composition according to claim 5.

9. The use of a compound according to claim 1 or a salt thereof with a pharmaceutically acceptable acid for the preparation of a medicament useful in treatment of calcium overload in brain cells of mammals, including humans.
